# EUROPEAN PATENT APPLICATION

(11) **EP 1 143 250 A2**
(43) Date of publication of application: **10.10.2001**
(21) Application number: 01303132.3
(22) Date of filing: 02.04.2001
(51) Int. Cl.: G01N 33/543, G01N 33/76, G01N 33/577, C07K 16/26

(54) **Test methods and devices for analyte isoforms**

(30) Priority: 03.04.2000 EP 00302810
(71) Applicant: UNILEVER PLC, London EC4P 4BQ (GB); UNILEVER N.V., 3013 AL Rotterdam (NL)
(72) Inventor: Butlin, Lorraine Diane, Sharnbrook, Bedford MK44 1LQ (GB); Coley, John, Sharnbrook, Bedford MK44 1LQ (GB); Eida, Stephen James, Sharnbrook, Bedford MK44 1LQ (GB); Gani, Mohamed Mutwahar, Sharnbrook, Bedford MK44 1LQ (GB)
(74) Representative: Evans, Jacqueline Gail Victoria

(57) **Abstract**

A method and test device for differentiating between states of an analyte that can exist in different forms, such as follicle stimulating hormone (FSH). The method or test device uses two contemporaneous assays, the first of which does not differentiate between the two analyte states and the second of which does, and the assay results are compared. A novel pair of anti-FSH monoclonal antibodies that can be used together in a sandwich-format assay to differentiate pre-menopausal and post-menopausal FSH samples is disclosed.

## Description

### Field of the Invention

This invention relates to test methods and devices, and more particularly to methods for differentiating between states of an analyte that exists as various forms, e.g. isoforms.

### Background to the Invention

Tests are available, or have been proposed, which purport to provide clinically significant information about hormonal levels of relevance to the menopause. The principal hormone of interest is follicle stimulating hormone (FSH). The post-menopausal state has been associated with a rise in the level of circulating FSH. For this purpose tests have been developed to detect the level of FSH in body fluid samples such as blood and urine. These tests are intended to detect "total" FSH, in the sense that they do not discriminate between different isoforms of FSH.

These known tests are used by clinicians in recommending and monitoring hormone replacement therapy (HRT). As the menopause is also associated with a drop in the level of circulating estrogen metabolites, HRT normally involves administration of estrogen in order to reduce this deficit and counteract symptoms associated with the menopause.

Although it is known that FSH exists in various forms, the clinical significance of these in relation to conditions such as the menopause is poorly understood. The differing forms may be isoforms or glycoforms. However, the existence of these differing forms calls into question the soundness of "total" FSH tests as a basis for good clinical diagnosis.

There is a need for an improved method of monitoring gonadotrophin hormones, especially FSH, to provide more reliable diagnosis of menopausal conditions and to facilitate the prescription and regulation of HRT.

More generally, there is a need for a method for differentiating between states of an analyte that exists as a plurality of forms, especially when the nature and/or relative amounts of such forms present in a sample of the analyte may be of clinical significance. The forms may differ from one another in either physical characteristics (e.g. "isoforms" separable by charge) or chemical characteristics (e.g. "glycoforms" in the case of FSH or similar molecules), or indeed both.

### General Description of the Invention

The invention provides a method for differentiating between two states of an analyte that exists in a plurality of forms, which states differ from one another in the nature and/or amount of one or more forms present therein, wherein:
a) at least two contemporaneous assays are conducted, the first of which has a specificity for the analyte that is essentially constant irrespective of whether the analyte is in one form or the other, and the second of which has a specificity for the analyte that differs depending on which form the analyte is in; and
b) the results of the first and second assays are compared.

In a preferred embodiment of the invention we provide a method of testing for the existence of a menopausal condition in a human female which involves two separate contemporaneous tests for a gonadotrophin, especially FSH. The first test does not differentiate between the gonadotrophin found in pre-menopausal and post-menopausal subjects. However, the second test does differentiate in this respect. The specificity of the second test for the gonadotrophin is different depending on whether the sample under test is derived from a pre-menopausal or post-menopausal subject. By conducting both tests at essentially the same time a reliable indication can be given both of the total level of circulating gonadotrophin and whether that gonadotrophin is of a type that can be associated with the menopausal state.

In particular the invention provides a method of monitoring the hormonal status of an individual human female subject in which the contemporaneous tests are conducted repeatedly, i.e. at regular intervals such as every few weeks, to determine whether the gonadotrophin level and its character are changing in a manner which indicates entry into or departure from a menopausal state.

In another embodiment, the invention provides a method of testing for the existence of a menopausal condition in a human female by means of a gonadotrophin assay, wherein:
a) at least two contemporaneous assays are conducted, the first of which has a specificity for the gonadotrophin that is essentially constant irrespective of whether the human female is pre-menopausal or post-menopausal, and the second of which has a specificity for the gonadotrophin that differs depending on whether the human female is pre-menopausal or post-menopausal; and
b) the results of the first and second assays are compared.

Another embodiment of the invention is an assay device for testing a body fluid sample obtained from a human female, the device having a first analyte-responsive (preferably gonadotrophin-responsive) signal-producing means that provides a readable signal that, relative to a reference standard, is constant irrespective of whether the sample if derived from a pre-menopausal or post-menopausal subject, and a second analyte-responsive (preferably gonadotrophin-responsive) signal-producing means that provides a readable signal that, relative to a reference standard, differs depending on whether the sample is derived from a pre-menopausal post-menopausal subject.

Each readable signal can be caused by the binding in a detection zone of a specific binding agent labelled with a direct particulate label, such as a gold sol or coloured latex particle. Alternatively, other signal-producing labels can be used, for example enzyme labels, fluorescent labels or radiolabels.

Desirably the specific binding agent comprises an antigen binding site of an immunoglobulin. The specific binding agent may conveniently be an antibody (e.g. an IgG or IgA molecule), or comprise any antigen binding portion thereof, such as Fv, Fab, scFv, bispecific antibodies, "diabodies" and the like, all of which are well known to those skilled in the art.

Particularly preferred binding agents are monoclonal antibodies.

The contemporaneous tests of the invention can be conducted repeatedly, generally at an interval of at least a week, to monitor the effectiveness of a course of HRT.

The sample tested in the contemporaneous assays may be a sample of any suitable body fluid from the subject, such as blood, serum, plasma, sweat, tears, crevicular fluid and the like. Most conveniently the sample is a sample of urine.

Although FSH is the preferred analyte for use in accordance with the invention, other members of the gonadotrophin family can be used. These include human chorionic gonadotrophin (hCG), luteinizing hormone (LH) and thyroid stimulating hormone (TSH). All of these gonadotrophins are glycopeptides. Their principal structure comprises two peptide chains. One peptide chain, known as the alpha chain, is common to all members of the family. The other peptide change, known as the beta chain, differs in each molecule. In addition, each molecule contains glycoprotein side chains. The detailed structure of these molecules is not completely understood. However it is believed that variations in the composition of the glycoprotein side chains give rise to different forms ("glycoforms") of each molecule. Those skilled in the art will appreciate that differences in the chemical properties of the glycoprotein side chains may also influence the physical properties (e.g. charge) of the overall molecule, such that different glycoforms may also constitute different isoforms. Thus, in the case of FSH for example, on present scientific knowledge it is believed that the alpha and beta peptide chains are the same in all FSH forms, but subtle differences occur in the glycoprotein side chains. It is believed that the relative proportions of the forms of FSH existing in the menopause state are different from those in the pre-menopause state.

Prior to this invention it was not appreciated that a specific binding assay could be developed which would differentiate between the FSH forms, to an extent sufficient to enable worthwhile detection of a menopausal state to be achieved.

In a preferred embodiment of the invention both assays are of the sandwich format. Each assay therefore requires two specific binding agents (e.g. antibodies), preferably one directed against the alpha chain and the other against the beta chain of the FSH molecule. The antibody pairs must be different. At least one member of each pair, e.g. the anti-beta antibody, must differ from the corresponding antibody in the other pair. However, this alone does not necessarily lead to the desired objective. We have found surprisingly that an enhanced degree of differentiation between pre-menopausal and post-menopausal FSH can be achieved if the members of the second antibody pair are both different from the members of the first antibody pair. Thus, between the two assays, it is preferred that both the capture antibody and the labelled antibody are different. In a preferred embodiment the invention therefore uses two sandwich-format immunoassays for FSH in which the antibodies are directed against the alpha and beta peptide chains of the molecule, but are exhibiting differences in specificity for certain forms of FSH caused by subtle changes in the glycoprotein side chains.

Antibody pairs appropriate for use in the invention can be identified by screening a range of anti-FSH antibody pairs against FSH samples obtained from pre-menopausal and post menopausal women. The first antibody pair is selected as being one in which the sandwich-format assay gives constant results, when compared with a reference standard, irrespective of whether pre-menopausal or post-menopausal FSH samples are used. The second antibody pair under the same circumstances gives rise to assay results which are consistently different depending on whether the FSH sample is pre-menopausal or post-menopausal.

A suitable source from which to select antibodies appropriate for the first antibody pair are commercially available antibodies which are recommended for use in "total" FSH assays.

In order to provide a source of antibodies from which to select the second antibody pair (which differentiate between analyte forms) it is desirable, although not essential, to raise a panel of antibodies against the analyte forms in question. This can be done by routine hybridoma technology or, alternatively, an immunoglobulin-producing bacteriophage library can be screened.

A particular aspect of the invention in relation to its application to the analysis of FSH samples is a pair of novel anti-FSH monoclonal antibodies that distinguish between pre-menopausal and post-menopausal FSH samples. Two murine hybridoma cell lines each expressing one of these novel monoclonal antibodies have been deposited in accordance with the provisions of the Budapest Treaty 1977 in the European Collection of Cell Cultures (ECACC, Centre for Applied Microbiology & Research, Salisbury, Wiltshire SP4 OJG, UK) as follows:
a) Balb/c murine hybridoma clone "4813.2" expressing an anti-beta-FSH monoclonal antibody: ECACC 00032004;
c) Balb/c murine hybridoma clone "4882.1" expressing an anti-alpha-FSH monoclonal antibody: ECACC 00032005, (both deposited at ECACC on 20^{th} March 2000).

The invention includes the use of either or both of the anti-FSH monoclonal antibodies as expressed by these deposited cell lines, in a method or analytical test device as set forth herein.

In practice the two assays should be performed on the same clinical sample, sub-divided if necessary, or on two samples obtained from the same individual subject at more or less the same time (i.e. on the same day, preferably in the same hour) so that the two assays give results that can fairly be compared with each other. It is in this sense that we regard the assays as being contemporaneous. For example, if the sample may be stably stored [e.g. by freezing at -20°C] between tests, then the contemporaneous assays may actually be performed at different times. Two assays may therefore be considered contemporaneous assays if they are performed on the same sample or separate aliquots of the same sample, or on separate samples obtained from the subject at more or less the same time. The results of the two contemporaneous assays are compared to determine whether a menopausal state exists.

In one embodiment the test results can be interpreted on a qualitative or semi-quantitative basis, for example by eye if the two assays give rise to visible test readings which can be interpreted readily, for example through differences in colour intensity. If necessary this visual determination can be aided by the provision of a reference standard. The two assays can be configured to aid visual assessment. For example, the performances of the two assays can be modulated such that in a non-menopausal state both assays give rise to a similar signal in terms of a particular colour or colour intensity, whereas in a menopausal state the second assay produces a discernably different colour or colour intensity.

For more accurate diagnosis of menopausal conditions it may be appropriate for the assay results to be determined numerically. This will usually require a sophisticated reading system, such as by optical transmission or reflectance and which is amenable to measuring small changes in signal intensity and relating these to FSH concentrations. In this situation it may be appropriate to determine the numerical ratio of the signals of the first and second assays. A significant change in this ratio can indicate transition from a pre-menopausal to a post-menopausal state, or vice-versa. Thus the results from a series of contemporaneous tests performed, for example, every few weeks, can be collated and any change in the observed signal ratio used to diagnose a change in condition.

For the purposes of HRT monitoring, the HRT treatment, either in terms of the therapeutic product used or its dose level, can be modulated to maintain the ratio value from successive double tests at a pre-determined level, for example.

Test devices using the assays of the invention can be provided for home use or for use in clinics or doctors' offices. Alternatively laboratory-style assays can be used. Preferred assay formats involve the single step format as described, for example, in EP-A-291194. These assays can be used if desired in combination with an electronic reader, for example as described in WO 95/13531. In this instance preferably the electronic reader has an information downloading facility, e.g. by means of a transferable datacard ("smart card"), from which a user, e.g. clinician, can transfer data to a computer during consultation with the patient, in order that stored information from repeated tests can be interpreted properly for diagnostic purposes. The computer can include programmed information that assists the clinician in establishing an appropriate HRT treatment for the individual subject.

Generally, the method of the invention involves the use of two different pairs of specific binding agents, each pair being used in one of the contemporaneous assays. The two assays will normally be of the same format, e.g. both heterogeneous sandwich-format assays, operating under as near identical conditions as possible, so that a difference in result between the two assays is attributable solely to the different binding agents used.

The following example illustrates aspects of the invention in greater detail.

### Example

### 1. Raising anti-FSH monoclonal antibodies

Balb/c mice were immunised with human FSH preparations, derived from urine and pituitary sources, purified by immuno-affinity prior to immunisation. Monoclonal antibodies were produced from the immunised mice using conventional cloning techniques, by fusing spleen cells with SP2/Ag14 cells as the immortal partner.
The subunit specificity of the anti-FSH monoclonal antibodies were assigned by means of anti-alpha subunit antibodies cross reactive with LH, TSH and hCG.

### 2. Identification of anti-FSH antibody pairs with fertile state bias

a) A panel of seven human urine samples were used to screen various antibody pairs. The samples from young fertile women (under 35 years of age with regular menstrual cycles) were taken at the (1)early follicular, (2)mid-follicular, (3)ovulation, (4)mid-luteal and (5)late luteal phases of menstrual cycles. For each fertile phase pooled samples from two individuals were used. The phase of the menstrual cycle was determined retrospectively by the urinary profiles of the hormones FSH,LH, E3G and P3G. In addition to the fertile samples, two post menopausal urine samples were used (6) 1 month before commencement of HRT treatment and (7)one taken at least one month after HRT treatment began.
   For use in the antibody screen the urine panel was normalised based on FSH concentration estimates. The FSH concentration estimates were obtained using commercially available anti-FSH monoclonal antibodies (Clone No's. 6601 and 6602 from Medix Biochemica, Finland) in a sandwich-format ELISA assay. Samples were normalised by being concentrated using centrifuge filtration.
b) Screening procedure
   1) FSH antibodies for screening were prepared at concentrations of 2.5µg/ml in 0.2M sodium carbonate buffer pH 8.0.
   2) 200µl of the antibody dilutions to be screened were added to wells in High binding Greiner 96-well microtitre plates, which were then incubated overnight at 37(C.
   3) The plates were washed three times in PBSTA.
   4) 100µl of 0.38M Tris was added into all of the plate wells, except the blanking wells to which PBSTA was added. 100µl of each urine sample from the screening panel (see paragraph a) were added at 15.3mIU/ml (based on the 6602/6601 assay estimates) to triplicate wells for each antibody/conjugate pairing.
   5) The plates were incubated for 1 hour at room temperature.
   6) Step 3 was repeated.
   7) 200µl of an optimum dilution in PBSTA of alkaline-phosphatase conjugated anti-beta FSH subunit antibody was added to wells sensitised with anti-alpha FSH subunit capture antibodies. 200µl of an optimum dilution in PBSTA of alkaline-phosphatase conjugated anti-alpha FSH subunit antibody was added to wells sensitised with anti-beta FSH subunit capture antibodies.
   8) As a control in each screening run the urine panel was tested with a reference assay (the "Medix assay") using Medix Biochemica clone No. 6601 conjugate paired with Medix Biochemica clone No.6602 as the capture antibody.
   9) Step 5 was repeated.
   10) Step 3 was repeated.
   11) 200µl of DEAE substrate was pipetted into all wells of all plates.
   12) Step 5 was repeated.
   13) The plates were read on a Dynatech plate reader at 405nm after 1hour 30 minutes incubation.
   14) The mean values of the triplicates were then calculated, and compared to the Medix assay O.D. values. This allowed antibody pairs showing bias in sample recognition relative to the Medix assay to be identified.

The two hydridoma cell lines referred to earlier, now deposited with the ECACC, were selected using this procedure.

### 3. Use of the FSH ratio for menopause confirmation

The method described in section 5 below was used to test urine samples from 8 cycles in each of 8 fertile women, and from 30 daily samples from each of 8 post-menopausal women.
The study compared the FSH assay ratio with total FSH measurement (using the reference assay) for the ability of the assay to differentiate between the urine of fertile and post-menopausal women.

The FSH assay ratio clearly differentiates between fertile and menopausal women.

### 4. Changes in FSH assay ratio associated with HRT treatment

Urine samples from a post-menopausal woman taken before and up to 6 weeks after HRT treatment were tested as described in section 3. It was found that the FSH assay rises above the level for post-menopausal after 2 weeks of the commencement of treatment. The ratio value rises with continued treatment until it reaches values obtained for fertile women.

### 5. Method used to assess menopause state using FSH ratio

1) Greiner high binding 96 well microtitre plates were sensitised with 200(1 per well of 5µg/ml streptavidin (Pierce code No.21125) in carbonate buffer pH 9.8 overnight at +4°C.
2) The FSH standard (Scipac FSH 9606 stock, FSH concentration = 99.6 ng/ml) was diluted to [FSH]= 10 ng/ml in PBS and nine doubling dilutions were made from this stock in the same buffer to give an FSH concentration range of 0.02 - 10 ng/ml. The urine samples from the fertile women were assayed neat and those from the peri- and post-menopausal women were assayed both neat and diluted 1/5 in PBS.
3) The streptavidin sensitised plates were washed three times with PBSTA using a plate washer.
4) 200 µl per well of 2.5 µg/ml biotinylated anti-FSH anti-beta subunit monoclonal antibodies clone No. 6602 or the capture antibody from the biased assay (Example 2) in PBSTA was added to the plates. The plates were incubated for 30 minutes at 37°C in a water bath.
5) The antibody sensitised plates were washed three times with PBSTA.
6) 30 µl per well of 2% W/V BSA in 2.3 M. Tris pH 8.0 was added to the plates.
7) 170µl of the test samples were added to duplicate wells on one 6602 and one biased assay plate. Also 170µl of the FSH standard dilutions were added to duplicate wells on each of the sensitised plates.
8) The plates were incubated for 1 hour at 37°C in a water bath.
9) Step 3 was repeated.
10) 200µl per well of a 1/600 dilution, in PBSTA, of alkaline phosphatase conjugated clone No.6601 anti-alpha FSH subunit monoclonal antibody was added to the 6602 sensitised plates. 200 µl per well of a optimum dilution, in PBSTA, of alkaline phosphatase conjugated partner antibody for the biased assay was added to the biased assay plates.
11) Step 8 was repeated.
12) Step 3 was repeated.
13) 200µl per well of alkaline phosphatase substrate was added to the plates.
14) Both sensitised plates were incubated at 37°C in a water bath until adequate colour had developed, and then the O.D. 405nm was read on a plate reader.
15) The total FSH concentration for each urine sample was calculated from both assays using the standard curve. The ratio of the biased assay to that of the reference assay was also calculated for each sample.

Standard buffer solutions used above:
- PBSTA:: Phosphate buffered saline
0.01M Phosphate pH 7.2
0.9 % Sodium chloride
0.15% Tween 20
0.02% Sodium azide
- PBSA:: As above, no Tween 20
- PBS:: As above, no Tween 20 or sodium azide

### Summary of FSH Assay Ratio Results

| Group | No of individuals tested | **Mean FSH assay ratio for group** |
|---|---|---|
| Fertile | 8 | **2.82** |
| Menopausal | 8 | **1.46** |

## Claims

1. A method for differentiating between two states of an analyte that exists in a plurality of forms, which states differ from one another in the nature and/or amount of one or more forms present therein, wherein:
a) at least two contemporaneous assays are conducted, the first of which has a specificity for the analyte that is essentially constant irrespective of whether the analyte is in one form or the other, and the second of which has a specificity for the analyte that differs depending on which form the analyte is in; and
b) the results of the first and second assays are compared.

2. A method of testing for the existence of a menopausal condition in a human female by means of a gonadotrophin assay, wherein:
a) at least two contemporaneous assays are conducted, the first of which has a specificity for the gonadotrophin that is essentially constant irrespective of whether the human female is pre-menopausal or post-menopausal, and the second of which has a specificity for the gonadotrophin that differs depending on whether the human female is pre-menopausal or post-menopausal; and
b) the results of the first and second assays are compared.

3. A method according to claim 1, wherein the analyte is a gonadotrophin.

4. A method according to claim 2 or 3, wherein the gonadotrophin is follicle stimulating hormone (FSH).

5. A method according to any one of the preceding claims, wherein both contemporaneous assays are sandwich-format assays.

6. A method, according to any one of claims 2-5, wherein each of said at least two contemporaneous assays uses an antibody pair directed against the alpha and beta peptide chains of the gonadotrophin, but both members of the antibody pair in the first assay differ from the members of the antibody pair in the second assay.

7. A method according to any one of the preceding claims, wherein each assay provides a quantitative result, and the ratio of the two results is taken as indicative of menopausal status..

8. A method according to any one of the preceding claims, wherein the at least two contemporaneous assays are repeated at intervals of at least one week to determine whether the menopausal status is changing.

9. A method according to claim 8, wherein the human subject is one undergoing a course of HRT.

10. An assay device for testing a body fluid sample obtained from a human female, the device having a first analyte-responsive (preferably gonadotrophin-responsive) signal-producing means that provides a readable signal that, relative to a reference standard, is constant irrespective of whether the sample is derived from a pre-menopausal or post-menopausal subject, and a second analyte-responsive (preferably gonadotrophin-responsive) signal-producing means that provides a readable signal that, relative to a reference standard, differs depending on whether the sample is derived from a pre-menopausal or post-menopausal subject.

11. An assay device according to claim 10, wherein the gonadotrophin is FSH.

12. An assay device according to claim 10 or claim 11, wherein each readable signal is caused by the binding in a detection zone of a specific binding agent labelled with a particulate direct label.

13. A method according to claim 1, or an assay device according to claim 10, substantially as hereinbefore described.

14. A method according to claim 4, or an assay device according to claim 11, wherein the first assay, or first gonadotrophin-responsive signal-producing means, as appropriate, uses a pair of anti-FSH antibodies that detect "total" FSH.

15. An anti-FSH monoclonal antibody as expressed by hybridoma cell line ECACC 00032004.

16. An anti-FSH monoclonal antibody as expressed by hybridoma cell line ECACC 00032005.

17. A method according to claim 4, or an assay device according to claim 11, wherein the second assay uses either or both of the anti-FSH antibodies claimed in claims 15 and/or 16.
